# EUROPEAN PATENT APPLICATION

(11) **EP 3 199 642 A1**
(43) Date of publication of application: **02.08.2017**
(21) Application number: 16153617.2
(22) Date of filing: 01.02.2016
(51) Int. Cl.: C12Q 1/68, A01H 1/04

(54) **PLANT BREEDING USING HIGH THROUGHPUT SEQUENCING**

(71) Applicant: Fraunhofer-Gesellschaft zur Förderung der angewandten Forschung e.V., 80686 München (DE)
(72) Inventor: Schröper, Florian, 50321 Brühl (DE); Fritsch, Leonie, 52066 Aachen (DE); Schillberg, Stefan, 52074 Aachen (DE)
(74) Representative: Roth, Andy Stefan

(57) **Abstract**

The technology provided herein relates to novel methods for screening/detecting of plants, in particular by a multiplex PCR-based combined with a next-generation sequencing approach to analyze a plurality of characteristics (e.g. target sequences) of an individual plant in parallel.

SEQUENCE LISTING

<110>  Fraunhofer-Gesellschaft zur Förderung der angewandten
       Forschung e.V.

<120>  PLANT BREEDING USING NEXT GENERATION SEQUENCING

<130>  FRA-PA22-EP

<160>  25

<170>  PatentIn version 3.5

<210>  1
<211>  61
<212>  DNA
<213>  Hordeum vulgare

<400>  1
ggaacgacgc cctcaggcgt caacgcggcc ggccatggcc ggccagggat ttcgcccgaa      60

c                                                                      61

<210>  2
<211>  44
<212>  DNA
<213>  Hordeum vulgare

<400>  2
ggaacgacgc cctcaggcgt caacgccagg gatttcgccc gaac                       44

<210>  3
<211>  61
<212>  DNA
<213>  Hordeum vulgare

<400>  3
ggaacgacgc cctcaggcgt caacgcggcc ggccatggcc ggccagggat ttcgcccgaa      60

c                                                                      61

<210>  4
<211>  61
<212>  DNA
<213>  Hordeum vulgare

<400>  4
ggaacgacgc cctcaggcgt caacgcggcc ggccatggcc ggccagggat ttcgcccgaa      60

c                                                                      61

<210>  5
<211>  44
<212>  DNA
<213>  Hordeum vulgare

<400>  5
ggaacgacgc cctcaggcgt caacgccagg gatttcgccc gaac                       44

<210>  6
<211>  63
<212>  DNA
<213>  Hordeum vulgare

<400>  6
cgaggtggac ctctacaagt tcgacccatg cgagctcccc ggtatgtact actagttagt      60

act                                                                    63


<210>  7
<211>  63
<212>  DNA
<213>  Hordeum vulgare

<400>  7
cgaggtggac ctctacaagt tcgacccatg ggagctcccc ggtatgtact actagttagt      60

act                                                                    63


<210>  8
<211>  63
<212>  DNA
<213>  Hordeum vulgare

<400>  8
cgaggtggac ctctacaagt tcgacccatg cgagctcccc ggtatgtact actagttagt      60

act                                                                    63


<210>  9
<211>  63
<212>  DNA
<213>  Hordeum vulgare

<400>  9
cgaggtggac ctctacaagt tcgacccatg ggagctcccc ggtatgtact actagttagt      60

act                                                                    63


<210>  10
<211>  63
<212>  DNA
<213>  Hordeum vulgare

<400>  10
cgaggtggac ctctacaagt tcgacccatg cgagctcccc ggtatgtact actagttagt      60

act                                                                    63


<210>  11
<211>  20
<212>  DNA
<213>  Artificial Sequence

<220>
<223>  Primer

<400>  11
ccagctgatg aaactccgaa                                                   20


<210>  12
<211>  20
<212>  DNA
<213>  Artificial Sequence

<220>
<223>  Primer

<400>  12
agcccatgtc agtttccagt                                                   20


<210>  13
<211>  20
<212>  DNA
<213>  Artificial Sequence

<220>
<223>  Primer

<400>  13
ggcgtatagt ctcggagtga                                                   20


<210>  14
<211>  20
<212>  DNA
<213>  Artificial Sequence

<220>
<223>  Primer

<400>  14
catttgcatc tgccgatatg                                                   20


<210>  15
<211>  20
<212>  DNA
<213>  Artificial Sequence

<220>
<223>  Primer

<400>  15
cttgaacggt atgagcgcta                                                   20


<210>  16
<211>  20
<212>  DNA
<213>  Artificial Sequence

<220>
<223>  Primer

<400>  16
caagcaccac ccaaccccaa                                                   20

<210>  17
<211>  20
<212>  DNA
<213>  Artificial Sequence

<220>
<223>  Primer

<400>  17
tacagaaccg acgacccaag                                                   20


<210>  18
<211>  20
<212>  DNA
<213>  Artificial Sequence

<220>
<223>  Primer

<400>  18
ctcaagaaga aggccgccaa                                                   20


<210>  19
<211>  20
<212>  DNA
<213>  Artificial Sequence

<220>
<223>  primer

<400>  19
tccacgcatc catccatcat                                                   20


<210>  20
<211>  20
<212>  DNA
<213>  Artificial Sequence

<220>
<223>  primer

<400>  20
gtatgtgatc ctgtcgtcgt                                                   20


<210>  21
<211>  20
<212>  DNA
<213>  Artificial Sequence

<220>
<223>  primer

<400>  21
gagaaggtcg gcgtcaagaa                                                   20


<210>  22

<211>  20
<212>  DNA
<213>  Artificial Sequence

<220>
<223>  primer

<400>  22
taacaggagc agaaacgtcg                                                    20


<210>  23
<211>  20
<212>  DNA
<213>  Artificial Sequence

<220>
<223>  primer

<400>  23
gtttccagca tcacgtccaa                                                    20


<210>  24
<211>  23
<212>  DNA
<213>  Artificial Sequence

<220>
<223>  primer

<400>  24
cctctctatg ggcagtcggt gat                                                23


<210>  25
<211>  30
<212>  DNA
<213>  Artificial Sequence

<220>
<223>  primer

<400>  25
ccatctcatc cctgcgtgtc tccgactcag                                         30

## Description

### FIELD OF THE DISCLOSURE

The present disclosure pertains to novel methods for screening/detecting of plants, in particular by a multiplex PCR-based combined with a next-generation sequencing approach to analyze a plurality of characteristics (e.g. target sequences) of an individual plant in parallel.

### BACKGROUND

Plant cultivars and varieties with yield, nutritional quality and agronomic performance optimized for different environments are needed to supply the growing global population (UN, 2008). Optimized characteristics can be achieved by conventional breeding, but this takes up to 8 years because phenotype-based testing requires fully grown plants (Borlaug, 1983, ISAAA, 2014). The speed and efficiency of plant breeding can be improved by adopting technologies such as reverse breeding, marker assisted selection (MAS) and genetic modification, all of which have advantages and disadvantages (He, Zhao, Laroche, Lu, Liu and Li, 2014, Jonas and de Koning, 2013, Nakaya and Isobe, 2012, Varshney, Nayak, May and Jackson, 2009). These modern techniques are steadily replacing or augmenting classical breeding approaches.

Barley for example is an important cereal crop which has been cultivated for thousands of years (Reets and Léon, 2004). It ranks fourth in terms of production volume behind maize, rice, and wheat, and is primarily used for food, feed and the production of alcoholic beverages (FAO, 2014). Barley is cultivated in different climates, soils and environments, and is exposed to diverse forms of abiotic and biotic stress.

The development of new sequencing instruments ("next generation" or "massively parallel") had a massive impact on genomics, since next generation sequencing (NGS) enables the generation of millions to hundreds of millions of reads in the same sequencing run. As a consequence, this technique has already found numerous applications in molecular and evolutionary biology, metagenomics, and clinical areas, such as in the analysis of genomes, in human genetics, forensics, prenatal screening, early detecting of cancer, etc.

Many NGS platforms differ in engineering configurations and sequencing chemistry. However, most sequencing approaches use an in vitro cloning step to amplify individual DNA molecules, because their molecular detection methods are not sensitive enough for single molecule sequencing. Thus, the recent sequencing platforms all share the technical paradigm of massive parallel sequencing via spatially separated, clonally amplified DNA templates or single DNA molecules in a flow cell. This design is very different from that of Sanger sequencing - also known as capillary sequencing or first-generation sequencing - which is based on electrophoretic separation of chain-termination products produced in individual sequencing reactions.

Next generation sequencing generates large amounts of data in a short time by producing thousands or even millions of reads in parallel. The increasing throughput and falling costs of NGS have encouraged multiple applications in different areas of the life sciences, including medicine (Metzker, 2010) and agriculture (Elshire, Glaubitz, Sun, Poland, Kawamoto, Buckler and Mitchell, 2011, Mascher, Wu, Amand, Stein and Poland, 2013, Teixeira, Fortes, Pinheiro and Pereira, 2014, You, Huo, Deal, Gu, Luo, McGuire, Dvorak and Anderson, 2011).

For example, the high-throughput sequencing of large numbers of amplicons has been used to genotype the human leukocyte antigen (HLA) locus (Bentley, Higuchi, Hoglund, Goodridge, Sayer, Trachtenberg and Erlich, 2009, Holcomb, Hoglund, Anderson, Blake, Bohme, Egholm, Ferriola, Gabriel, Gelber, Goodridge, Hawbecker, Klein, Ladner, Lind, Monos, Pando, Proll, Sayer, Schmitz-Agheguian, Simen, Thiele, Trachtenberg, Tyan, Wassmuth, White and Erlich, 2011) and to determine zygosity in transgenic maize (Fritsch, Fischer, Wambach, Dudek, Schillberg and Schröper, 2015).

EP 2 200 424 B1 discloses a method for the selection of a population of plants that have an artificial mutation in a desired genomic area that may lead to improved genetic variation and improved phenotypes. After cultivating the plants in a defined order, the genomic DNA is isolated, pooled and parts of the desired genomic area comprising the inserted mutation is amplified. After the amplification, the amplicons will be sequenced and compared with a reference sequence. The several plant pools may be identified with a barcode sequence. Furthermore, EP 1 929 039 B2 discloses a high-throughput screening method for the detection of specific mutations in a plant population using next generation sequencing methods.

However, it is an object of the present disclosure to provide novel and improved methods for screening and/or selecting plants.

### SUMMARY OF THE DISCLOSURE

The present disclosure pertains to novel methods for selecting/detecting a plant from a plant population by genotyping, in particular for the use of plant breeding.

The present disclosure pertains to novel methods for plant selection useful for plant breeding processes, in particular by a multiplex PCR-based approach to analyze a plurality of characteristics (e.g. target sequences) of individual plants in parallel. First after the amplification, the amplification products (amplicons) of a plurality of plants are pooled and sequenced together by new sequencing instruments /techniques ("next generation" or "massively parallel"). Due to the use of barcode sequences a plurality of plants can be examined in parallel.

The methods according to the present disclosure are rapid and high-throughput analysis methods useful for plant breeders and farmers. These methods provide robust genotyping data that allow the rapid determination of genotype and zygosity. These methods can be used to genotype large panels of plants because up to 80 million individual reads can be produced in one sequencing run, and samples from different lines and/or traits can be pooled after the amplification step. These findings are significant because plant breeders may need to screen large populations for multiple traits in parallel. The methods provide further a simple and inexpensive approach for the rapid and accurate genotyping of natural polymorphisms e.g. in barley, which can also be applied in many other economically relevant crop species.

One advantage of the methods according to the present disclosure is that the amplification products could be allocated to an individual plant and to a reference sequence. Due to the distribution of the specific sequencing reads the presence or absence of specific alleles can be determined. Therefore, it can be identified if a characteristic is homozygous or heterozygous.

A further advantage of the methods of the present disclosure is the use of barcodes comprised in the amplicons of the individual plants. In contrast to the above-mentioned prior art, the isolated genomic DNA is not pooled before amplification. The individual amplicons can be marked up with the barcodes during amplification because the barcode is attached to the end of at least one of the primers used to amplify the polymorphism of interest. A multiplex PCR-based approach is used to analyze a plurality of characteristics (e.g. target sequences) in parallel. Thereby, the same barcode is used for the amplification of every polymorphisms in one specific plant/sample. A further advantage of the methods according to the present disclosure is the possibility for a statement relating to the allele distribution of an individual plant and therefore to determine the zygosity state of the plant.

Therefore, the present disclosure pertains to novel methods for selecting a plant from a plant population by genotyping, the method comprises
a) isolating genomic DNA of individual plants or individual plant seeds separately, to provide separate DNA samples;
b) amplifying a desired target sequence of said DNA samples with target-specific primers, wherein each target-specific primer comprises a target-specific hybridization sequence and an adapter sequence, wherein at least one of the primers contains a barcode sequence, whereby the resulting amplification products (amplicons) comprise the target sequence, the adapter sequences and the barcode sequence;
c) pooling the amplicons of step (b) to prepare an amplicon library;
d) sequencing said amplified target sequence by using a next generation sequencing (NGS) technique;
e) comparing the target-sequence with a known sequence of said target-sequence, wherein the target-sequence can be allocated to an individual plant by the barcode sequence.

In a second aspect, the present disclosure pertains to methods for screening a plant and/or a plant population for multiple characteristics by genotyping, the method comprises
a) isolating genomic DNA of individual plants or individual plant seeds separately, to provide separate DNA samples;
b) amplifying in parallel a plurality of different desired target sequences of said DNA samples with target-specific primers either separately or in a multiplex PCR reaction, wherein each target-specific primer comprises a target-specific hybridization sequence and an adapter sequence, wherein at least one of the primers contains a barcode sequence, whereby the resulting amplification products (amplicons) comprise the target sequence, the adapter sequences and the barcode sequence;
c) pooling the amplicons of step (b) to prepare an amplicon library;
d) sequencing said amplified target sequences by using a next generation sequencing (NGS) technique;
e) comparing the target-sequences with known sequences of said target-sequences, wherein the target-sequences can be allocated to an individual plant by the barcode sequences.

In one aspect, the present disclosure relates to a process for breeding plants using a method according to the present disclosure.

In a further aspect, the present disclosure pertains to a process for breeding plants which comprises growing plants of a species in an array of containers charged with growth medium of uniform characteristics in an environment of controlled climatic conditions with controlled supply of nutrients and feed water and changing the positions of the containers within the environment as required to ensure at least substantially uniform exposure of all plants in the containers to conditions in the environment, and which process further comprises the step of selecting plants for further breeding or for commercial use by using a method according to the present disclosure.

Before the disclosure is described in detail, it is to be understood that the terminology used herein is for purposes of describing particular embodiments only, and is not intended to be limiting. It must be noted that, as used in the specification and the appended claims, the singular forms "a," "an" and "the" include singular and/or plural reference unless the context clearly dictates otherwise. It is moreover to be understood that, in case parameter ranges are given which are delimited by numeric values, the ranges are deemed to include these limitation values.

### BRIEF DESCRIPTION OF THE DRAWINGS

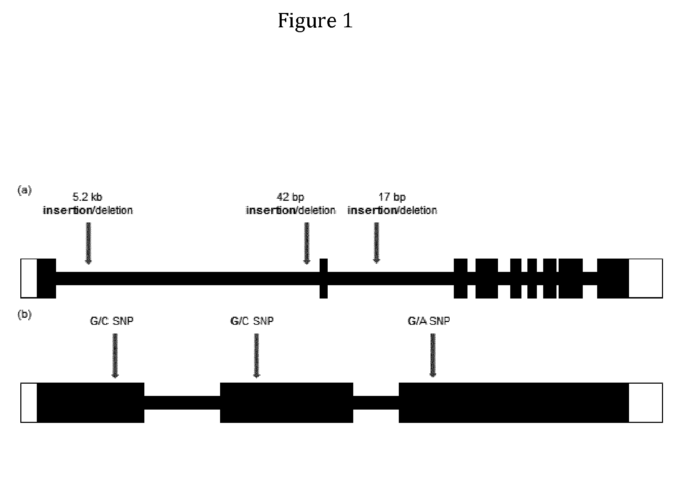

Figure 1: (a) Schematic depiction of the VrnH1 gene. The genotypic features of the winter barley line Strider are shown in bold, whereas normal font shows the genotypic features of the spring barley line Morex. (b) Schematic depiction of the HvNAM1 gene. The genotypic features of the low grain protein content barley line Karl are shown in bold, whereas normal font shows the genotypic features of the high grain protein content barley line Clipper. Horizontal bars show stretches of DNA with thick black bars representing exons, thin black bars representing introns and the thick white bars representing untranslated regions. The polymorphisms are indicated by arrows.

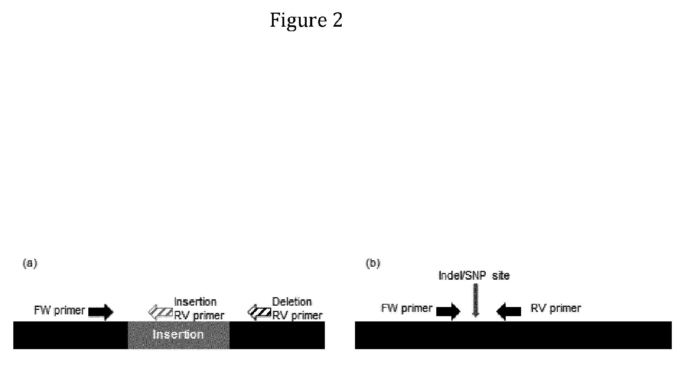

Figure 2: PCR strategies to amplify polymorphisms of interest. Horizontal bars show the DNA, horizontal arrows represent primers and the direction of elongation. (a) PCR strategy to amplify the flanking sequence of the 5.2-kb insertion at the VrnH1 locus. The striped arrows illustrate the use of two distinct reverse primers in a competitive PCR. (b) PCR strategy to amplify the small Indels in the VrnH1 locus and the SNPs in the HvNAM1 locus.

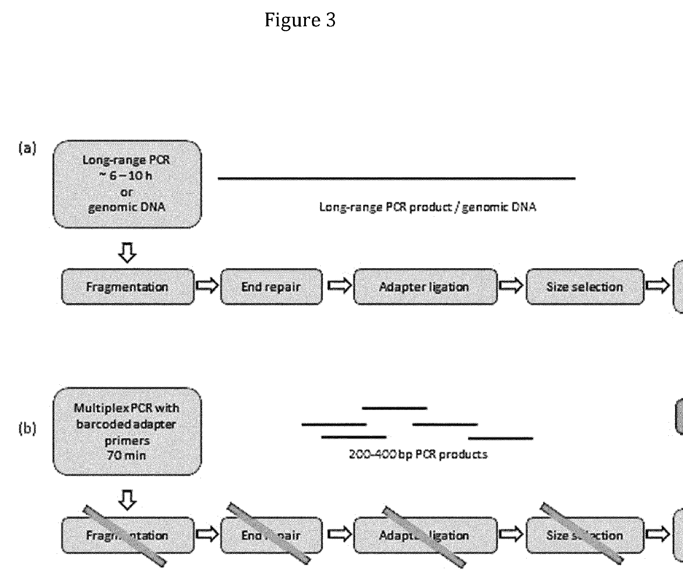

Figure 3: Schematic depiction and comparison of the workflow with either (a) genomic DNA/long PCR products or (b) short PCR products of 200-400 bp with attached barcodes and adapters. Working steps are shown as required to apply next-generation sequencing to the samples of interest, based on the designated PCR strategy and starting material. The crossed-out steps in (b) are omitted when PCR is carried out with barcoded primers amplifying 200-400-bp fragments.

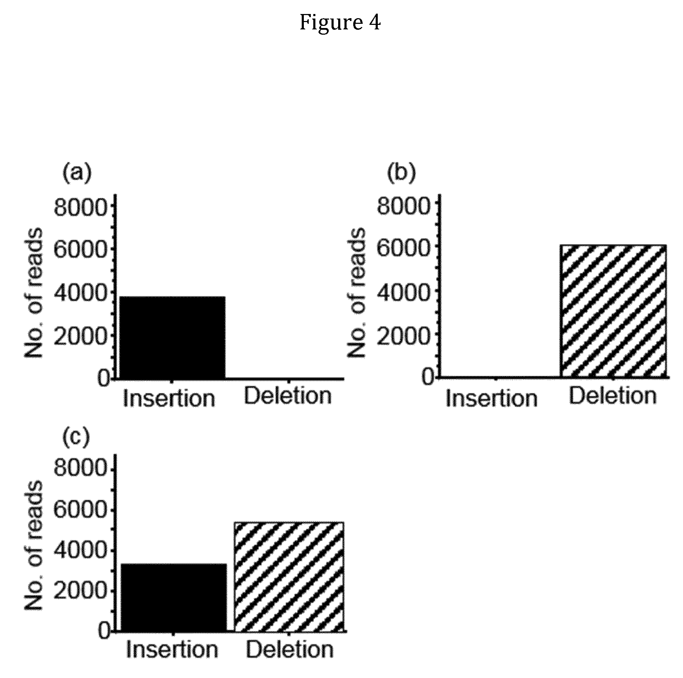

Figure 4: Allelic identification at the 5.2-kb Indel site of the VrnH1 gene, showing the number of reads aligned to the 5' sequence of the insert, or to the flanking sequence indicating a deletion. (a) Reads on the Strider template. (b) Reads on the Morex template. (c) Reads on the heterozygous Morex x Strider template. Insert: Reads aligned to the 5'-insert sequence. Deletion: Reads aligned to the sequence flanking the insertion site.

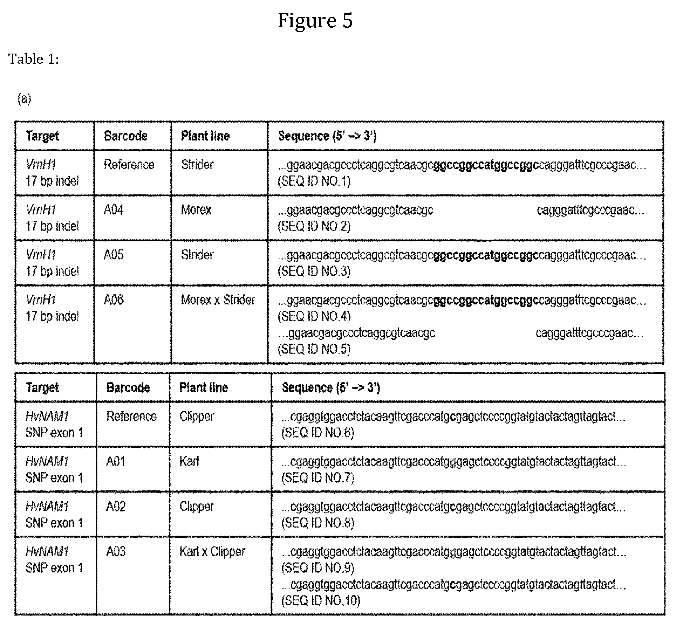

Figure 5: Sequencing results for (a) the 17-bp Indel in the VrnH1 gene and (b) the SNP at nucleotide position 243 in exon 1 for the HvNAM1 gene. The polymorphisms and flanking sequences are aligned to show the different alleles as well as their zygosity. Polymorphic sites are highlighted using bold letters.

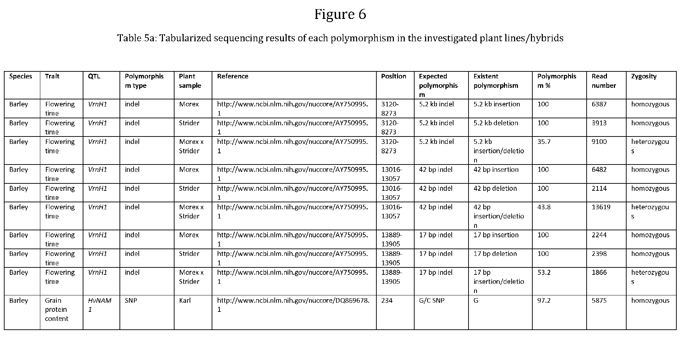

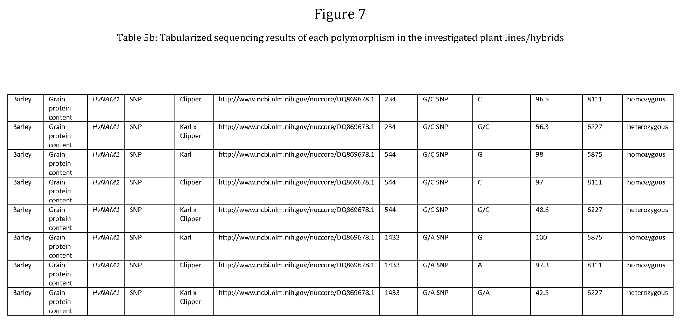

Figure 5 and 6 showing the tabularized sequencing results of each polymorphism in the investigated plant lines/hybrids

### DETAILED DESCRIPTION OF THIS DISCLOSURE

The present disclosure pertains to novel methods for screening/detecting of plants, in particular by a multiplex PCR-based approach to analyze a plurality of characteristics (e.g. target sequences) within an individual plant in parallel. First after the amplification, the amplification products (amplicons) of a plurality of plants are pooled and sequences together by new sequencing instruments /techniques ("next generation" or "massively parallel"). Due to the use of barcode sequences for each amplification product from the multiplex PCR, a plurality of plants can be examined in parallel.

The present disclosure pertains to novel methods for selecting a plant from a plant population by genotyping, the method comprises
a) isolating genomic DNA of individual plants or individual plant seeds separately, to provide separate DNA samples;
b) amplifying a desired target sequence of said DNA samples with target-specific primers, wherein each target-specific primer comprises a target-specific hybridization sequence and an adapter sequence, wherein at least one of the primers contains a barcode sequence, whereby the resulting amplification products (amplicons) comprise the target sequence, the adapter sequences and the barcode sequence;
c) pooling the amplicons of step (b) to prepare an amplicon library;
d) sequencing said amplified target sequence by using a next generation sequencing (NGS) technique;
e) comparing the target-sequence with a known sequence of said target-sequence, wherein the target-sequence can be allocated to an individual plant by the barcode sequence.

In advantageous embodiments, several different target sequences are amplified simultaneously in a multiplex PCR-based approach to analyze a plurality of characteristics (e.g. target sequences) in parallel, in particular in the separate sample.

Therefore, in some advantageous embodiments, with the method according to the present disclosure a plurality of individual plants are genotyped in parallel, and wherein the genomic DNA of a plurality of individual plants are isolated and amplified without pooling the genomic DNA before amplifying.

In a further embodiment, the methods according to the present disclosure the method comprises the steps of
a) isolating genomic DNA of individual plants or individual plant seeds separately, to provide separate DNA samples;
b) amplifying in parallel a plurality of different desired target sequences of said DNA samples with target-specific primers either separately or in a multiplex PCR reaction, wherein each target-specific primer comprises a target-specific hybridization sequence and an adapter sequence, wherein at least one of the primers contains a barcode sequence, whereby the resulting amplification products (amplicons) comprise the target sequence, the adapter sequences and the barcode sequence;
c) pooling the amplicons of step (b) to prepare an amplicon library;
d) sequencing said amplified target sequences by using a next generation sequencing (NGS) technique;
e) comparing the target-sequences with known sequences of said target-sequences, wherein the target-sequences can be allocated to an individual plant by the barcode sequences.

Therefore, in another advantageous embodiments, the present disclosure relates also to methods for screening a plant and/or a plant population for multiple characteristics by genotyping, the method comprises
a) isolating genomic DNA of individual plants or individual plant seeds separately, to provide separate DNA samples;
b) amplifying in parallel a plurality of different desired target sequences of said DNA samples with target-specific primers either separately or in a multiplex PCR reaction, wherein each target-specific primer comprises a target-specific hybridization sequence and an adapter sequence, wherein at least one of the primers contains a barcode sequence, whereby the resulting amplification products (amplicons) comprise the target sequence, the adapter sequences and the barcode sequence;
c) pooling the amplicons of step (b) to prepare an amplicon library;
d) sequencing said amplified target sequences by using a next generation sequencing (NGS) technique;
e) comparing the target-sequences with known sequences of said target-sequences, wherein the target-sequences can be allocated to an individual plant by the barcode sequences.

The plant population from which the genomic DNA is isolated may be a non-mutagenized population, mutagenized or transgenic plant population and the progeny thereof (including but not limited to plants or plant cells). The population may be plants, plant cells or plant seeds. The plants may be, for example, a grain crop, oilseed crop, fruit crop, vegetable crop, a biofuel crop, an ornamental plant, a flowering plant, an annual plant or a perennial plant. Examples of plants include but are not limited to petunia, tomato (Solanum lycopersicum), pepper (Capsicum annuum), lettuce, potato, onion, carrot, broccoli, celery, pea, spinach, impatiens, cucumber, rose, sweet potato, apple and other fruit trees (such as pear, peach, nectarine, plum), eggplant, okra, corn, soybean, canola, wheat, oat, rice, maize, sorghum, cotton and barley. In certain embodiments, the population is a variety of annuals. In specific embodiments, the population is a population of barley plants.

A technology that generates and uses mutagenized populations is known as TILLING (Targeted Induced Local Lesions In Genomes) (McCallum et al, Nat. Biotechnol 2000, 18, 455-457, McCalmm et al, Plant Physiology, -2000, 123, 439-442; Till et al Genome Research 2003, 13, 524-530) relies on random introduction of large numbers of mutations (mostly nucleotide substitutions) into the genome by treatment with ethyl methane sulfonate (EMS) or by ionizing radiation (fast neutron bombardment,) (Li et al The Plant Journal, 2001, 27, 235-42). Every plant in the population carries several hundred (or thousand) mutations, some of which affect normal development, morphology or otherwise confer a phenotype due to loss-of-function (knock-out, knock-down) of one or multiple genes or their regulatory sequences. A TILLING population generally contains a sufficient number of plants to cover all genes with multiple independent mutations (5-20 per gene).

TILLING" or "Targeting induced local lesions in genomes" is a general reverse genetic strategy providing an allelic series of induced (point) mutations by random chemical or physical mutagenesis in combination with PCR-based screening to identify point mutations in a region of interest. In TILLING screening, regions of interest are amplified by PCR. Heteroduplexes between wild-type fragments and fragments harboring an induced mutation are formed by denaturing and reannealing PCR products. These heteroduplexes are cleaved by CEL I and cleaved products are resolved. Throughput can be increased by pooling. Following discovery of PCR products harboring sequence differences in a pool, PCR products included in the pool are commonly screened again by Sanger sequencing of individual PCR products, thereby identifying the mutant plant and the exact sequence difference in the mutated gene.

"Mutagenized Population" refers to a population of plants, plant cells or plant seeds that have been subjected to mutagenesis (chemical or physical) to yield a library of mutants. TILLING plant populations may vary widely in size, and for certain purposes, partial TILLING populations can be used that contain 90, 80 70, 60, 50, 40 30 or even only 20% of the original population. As an alternative to mutagenized populations, populations can be used wherein the population is not mutagenized but comprises sub-populations that contain naturally occurring mutations such as Single nucleotide polymorphisms (SNPs), small insertions and deletions, and variations in microsatellite repeat number.

As used herein, "genotyping" means the identification of a genotype as a genetic component of the phenotype and it can be indirectly characterized using markers or directly characterized by nucleic acid sequencing. Suitable markers include a phenotypic character, a metabolic profile, a genetic marker, or some other type of marker. A genotype may constitute an allele for at least one genetic marker locus or a haplotype for at least one haplotype window. In some embodiments, a genotype may represent a single locus and in others it may represent a genome-wide set of loci. In another embodiment, the genotype can reflect the sequence of a portion of a chromosome, an entire chromosome, a portion of the genome, and the entire genome.

In a first step, genomic DNA of an individual plant (or individual plant seed) is isolated separately, to provide an individual DNA sample of each plant to be screened. Separate DNA samples means that the isolated genomic DNA of each individual plant in the plant population is not pooled after isolation and before the amplification step.

### Isolation of genomic DNA

The isolation of DNA is generally achieved using common methods in the art such as the collection of tissue from a member of the population, DNA extraction, quantification and normalization to obtain equal amounts of DNA per sample. A worker skilled in the art would readily appreciate that the quality of the genomic DNA as such, protocols which produce high quality genomic DNA with minimal contamination are preferable. In addition, a worker skilled in the art would readily appreciate that kits for isolation of genomic DNA are commercially available (for example Purelink™ Genomic Kit from Invitrogen or Wizard@ Genomic DNA Purification Kit from Promega).

### Amplification

In a second step, a desired target sequence or a plurality of desired target sequences comprised in the isolated genomic DNA is amplified by using an amplification technique known to a skilled artisan.

Presently, and as generally understood, the term "amplifying" or "amplification" in the context of nucleic acids refers to the production of multiple copies of a polynucleotide, or a portion of the nucleic acid molecule/polynucleotide, typically starting from a small amount of the polynucleotide (e.g., a single polynucleotide molecule), where the amplification products or "amplicons" are generally detectable. As such, a polymerase chain reaction represents one type of amplification reactions where a pair of primers is used that flank a desired target sequence. In conventional PCR the primers are mixed with a solution containing the target sequence (the template), a thermostable DNA polymerase and deoxynucleoside triphosphates (dNTPs). The reaction mixture is then heated to a temperature sufficient to separate the two complementary strands of the DNA template, and subsequently cooled to a temperature sufficient to allow the primers to specifically anneal to sequences flanking the gene or sequence of interest.

The "target sequence" or also called "target sequence of interest" may be coupled with a specific phenotype. In some embodiments in the present disclosure, the target sequence is selected from the group consisting of a polynucleotide, a nucleic acid pattern and a genomic region.

As used herein, "phenotype" means the detectable characteristics of a plant cell or plant that can be influenced by gene expression. The target sequence may be located at a specific locus. A "locus" is a position on a genomic DNA sequence that is usually found by a point of reference; e.g., a short DNA sequence that is a gene, or part of a gene or intergenic region. A locus may refer to a nucleotide position at a reference point on a chromosome, such as a position from the end of the chromosome. The ordered list of loci known for a particular genome is called a genetic map. A variant of the DNA sequence at a given locus is called an allele and variation at a locus, i.e., two or more alleles, constitutes a polymorphism. The polymorphic sites of any nucleic acid sequence can be determined by comparing the nucleic acid sequences at one or more loci in two or more germplasm entries.

Nucleic acid patters like DNA patters may serve as biological markers related to important life processes. A DNA pattern has a unique sequence such that it can be distinguished from the DNA patterns of other individuals. Differences in the sequence patterns between two samples can be due to inherited variations in the DNA that can distinguish two different samples. Prominent DNA patterns are gene networks, important motifs, spectrally and structurally repetitive DNA elements such as CpG islands, Alu repeats, non-coding RNAs (e.g., microRNAs and small nucleolar RNAs), tandem repeats, various type of satellite repeats, and the like. The methods according to the present disclosure may be employed to identify and/or locate for example repetitive elements in a variety of biologic systems, e.g., within a chromosome, within a genome, or across genomes of various species. Further examples for a nucleic acid pattern may be changed nucleic acids like Single Nucleotide Polymorphisms (SNPs), insertions or deletions of different length. Some examples of a nuclei acid pattern comprises a plurality (at least two) nucleic acid sequences e.g. two genes with different loci.

An example of a nucleic acid pattern is a Quantitative Trait Loci (QTL). As used herein, "quantitative trait locus (QTL)" means a locus that controls to some degree numerically representable traits that are usually continuously distributed. For example with the methods according to the present disclosure, different parts of a QTL could be amplified simultaneously with the multiplex approach.

In some advantageous embodiments, the target sequence is a chromosomal segment that comprises a portion of a natural and/or artificial genetic rearrangement like an inversion, insertion, deletion, or translocation. The target may comprise a (synthetic) mutagenic or DNA damaging oligonucleotide or, i.e. by Targeted Nucleotide Exchange (TNE) or by Region Targeted Mutagenesis (RTM), or populations that contain naturally occurring mutations such as Single nucleotide polymorphisms (SNPs), small insertions and deletions, and variations in microsatellite repeat number could be efficiently screened for the presence of mutations of interest. In an advantageous embodiment, the target sequence comprises a polymorphism like a Single Nucleotide Polymorphism (SNP).

As used herein, "polymorphism" means the presence of one or more variations of a nucleic acid sequence at one or more loci in a population of one or more individuals. The variation may comprise but is not limited to one or more base changes, the insertion of one or more nucleotides or the deletion of one or more nucleotides. A polymorphism may arise from random processes in nucleic acid replication, through mutagenesis, as a result of mobile genomic elements, from copy number variation and during the process of meiosis, such as unequal crossing over, genome duplication and chromosome breaks and fusions. The variation can be commonly found, or may exist at low frequency within a population, the former having greater utility in general plant breeding and the latter may be associated with rare but important phenotypic variation. Useful polymorphisms may include single nucleotide polymorphisms (SNPs), insertions or deletions in DNA sequence (Indels), simple sequence repeats of DNA sequence (SSRs) a restriction fragment length polymorphism, and a tag SNP. A genetic marker, a gene, a DNA-derived sequence, a haplotype, a RNA-derived sequence, a promoter, a 5' untranslated region of a gene, a 3' untranslated region of a gene, microRNA, siRNA, a QTL, a satellite marker, a transgene, mRNA, ds mRNA, a transcriptional profile, and a methylation pattern may comprise polymorphisms. In addition, the presence, absence, or variation in copy number of the preceding may comprise a polymorphism. As used herein, the term "single nucleotide polymorphism," also referred to by the abbreviation "SNP," means a polymorphism at a single site wherein said polymorphism constitutes a single base pair change, an insertion of one or more base pairs, or a deletion of one or more base pairs.

The target sequences serve as a useful tool for fingerprinting plants to inform the degree of identity of lines or varieties (US Patent 6,207,367). These markers form the basis for determining associations with phenotype and can be used to drive genetic gain. The implementation of marker-assisted selection is dependent on the ability to detect underlying genetic differences between individuals. Genetic markers for use in the present invention include "dominant" or "codominant" markers. "Codominant markers" reveal the presence of two or more alleles (two per diploid individual). "Dominant markers" reveal the presence of only a single allele. The presence of the dominant marker phenotype (e.g., a band of DNA) is an indication that one allele is present in either the homozygous or heterozygous condition. The absence of the dominant marker phenotype (e.g., absence of a DNA band) is merely evidence that "some other" undefined allele is present. In the case of populations where individuals are predominantly homozygous and loci are predominantly dimorphic, dominant and codominant markers can be equally valuable. As populations become more heterozygous and multiallelic, codominant markers often become more informative of the genotype than dominant markers.

Accordingly, the expression "amplification reaction" as presently used is meant to designate a reaction amplifying a piece of DNA. In the case of PCR reaction, this consists of cycles of repeated heating and cooling of a reaction mixture for DNA melting and enzymatic replication of the DNA. Key components in a PCR amplification reaction are primers, i.e. short DNA fragments containing sequences complementary to a target region of the DNA, and a DNA polymerase, which allow for a selective and repeated amplification. As PCR amplification progresses, the DNA generated is itself used as a template for replication, setting in motion a chain reaction in which the DNA template is exponentially amplified.

Accordingly, an amplification reaction consists of a first round of a PCR reaction comprising several cycles of cooling and heating of a PCR reaction mixture. Typically, one PCR "cycle" consists of a series of between 20 to 80 repeated temperature changes, with each cycle commonly consisting of 2 to usually 3 discrete temperature steps. The cycling is often preceded by a single temperature step at a high temperature (>90°C), and followed by one hold at the end for final product extension or brief storage. The temperatures used and the length of time that are applied in each cycle depend on a variety of parameters, including the enzyme used for DNA synthesis, the concentration of divalent ions and dNTPs in the reaction, and the melting temperature (Tm) of the primers.

As used herein, the term "primer" refers to an oligonucleotide that is capable of acting as a point of initiation of synthesis when placed under conditions in which synthesis of a primer extension product that is complementary to a nucleic acid strand is induced (e.g., in the presence of nucleotides and a DNA polymerase or the like, and at a suitable temperature and pH).

In some embodiments, the target sequence comprises a target portion and a flanking portion wherein the target-specific hybridization sequence in the primer may be complementary to a flanking portion and/or the target-specific hybridization sequence may be complementary to parts of the target portion.

As and when used herein, the term "nucleobase" is synonymous with other terms in use in the art including "nucleotide," "deoxynucleotide," "nucleotide residue," "deoxynucleotide residue," "nucleotide triphosphate (NTP)," or deoxynucleotide triphosphate (dNTP). As is used herein, a nucleobase includes natural and modified residues, as described herein.

An "oligonucleotide" refers to a nucleic acid that includes at least two nucleic acid monomer units (e.g., nucleotides), typically more than three monomer units, and more typically greater than ten monomer units. The exact size of an oligonucleotide generally depends on various factors, including the ultimate function or use of the oligonucleotide. Presently, the expressions "primer" or "oligonucleotide" or "oligonucleotide primer" are used to designate an oligonucleotide functioning as a primer as defined above.

As used herein, the terms "complementary" or "complementarity" are used in reference to polynucleotides (i.e., a sequence of nucleotides) related by the base-pairing rules. For example, the sequence "5'-A-G-T-3', is complementary to the sequence "3 -T-C-A-5'." Complementarity may be "partial," in which only some of the nucleic acids' bases are matched according to the base pairing rules. Or, there may be "complete" or "total" complementarity between the nucleic acids. The degree of complementarity between nucleic acid strands has significant effects on the efficiency and strength of hybridization between nucleic acid strands. This is of particular importance in amplification reactions, as well as detection methods that depend upon binding between nucleic acids.

The term "amplification product" or "amplicon" is used herein to designate products, which are produced by the extension of a primer. The products are at least partially double stranded, for example, in the region comprising the primer extension product and its complement. Accordingly, "double stranded" or at least partially double-stranded amplification products can be generated with an amplification reaction.

The definitions and methods provided define the present invention and guide those of ordinary skill in the art in the practice of the present invention. Unless otherwise noted, terms are to be understood according to conventional usage by those of ordinary skill in the relevant art. Definitions of common terms in molecular biology may also be found in Alberts et al., Molecular Biology of The Cell, 5th Edition, Garland Science Publishing, Inc.: New York, 2007; Rieger et al., Glossary of Genetics: Classical and Molecular, 5th edition, Springer- Verlag: New York, 1991; King et al, A Dictionary of Genetics, 6th ed, Oxford University Press: New York, 2002; and Lewin, Genes IX, Oxford University Press: New York, 2007. The nomenclature for DNA bases as set forth at 37 CFR $ 1.822 is used.

As mentioned above, a method of achieving such amplification employs the polymerase chain reaction (PCR) (Mullis et al. 1986 Cold Spring Harbor Symp. Quant. Biol. 51:263-273; European Patent 50,424; European Patent 84,796; European Patent 258,017; European Patent 237,362; European Patent 201,184; U.S. Patent 4,683,202; U.S. Patent 4,582,788; and U.S. Patent 4,683,194), using primer pairs that are capable of hybridizing to the proximal sequences that define a polymorphism in its double-stranded form.

In an advantageous embodiment, each individual (which means derived from one individual plant) genomic DNA is used as a template for polymerase chain reactions (PCR) which produce amplicons for one or more target sequence(s) comprised in the isolated genomic DNA.

The desired target sequence comprised in the isolated genomic DNA (DNA samples) may be amplified with target-specific primers separately or alternatively several target sequences in a multiplex PCR reaction, wherein each target-specific primer comprises a target-specific hybridization sequence, an adapter sequence and a barcode sequence, wherein the resulting amplification products (amplicons) comprise therefore a target sequence, an adapter sequence and a barcode sequence.

By using the above-mentioned primers, adapters are coupled at the 5' and/or 3' ends of the amplified DNA fragments, preferably at both ends of the obtained fragments. The specific design of the adapters depends on the next generation sequencing platform to be used and for the purposes of the present disclosure, basically any adaptors used for preparing sequencing libraries for next generation sequencing can be used. For example, the adaptors can be specified as P1 and A.

The adapter sequences provide a known sequence composition allowing e.g. subsequent library amplification and/or sequencing primer annealing. As adaptors, double-stranded or partially double-stranded nucleic acids of known sequence can be used. The adapters may have blunt ends, cohesive ends with 3' or 5'overhangs, may be provided by Y shaped adapters or by stem-loop shaped adapters. Y shaped adapters are e.g. described in US7,741 ,463 and stem-loop shaped adapters are e.g. described in US2009/0298075, herein incorporated by reference regarding the specific design of the adapters.

Preferably, the adaptors have a length of at least 7, preferably at least 10, preferably at least 15 bases. The adapter length preferably lies in a range of 10 to 100 bases, preferably 15 to 75 bases, more preferred 20 to 60 bases. Either the same or different adaptors can be used at the 3' and 5' end of the fragments. Using the same type of adaptor for both ends, such as e.g. a Y shaped or a stem-looped shaped adapter, has the advantage that no fragments are lost during library preparation due to adapter mispairing which is an advantage when working with low amounts of DNA.

Thus, preferably, the sequencing library used in the present disclosure consists double stranded DNA molecules comprising at their 3' and 5' end adapter sequences. The adapters provide a known sequence and thus provide a known template for amplification and/or sequencing primers.

Optionally, the adapters may also provide an individual index thereby allowing the subsequent pooling of amplicons derived from a plurality of individual plants, plant cells or plant seeds to prepare an amplicon library prior to sequencing. This embodiment will be described in further detail below. Suitable methods for preparing sequencing libraries are also described in Metzker, 201 1, Voelkerding, 2009, and WO12/003374. As described above, depending on the NGS technology used, several thousands, several millions or even up to billions of reads per run can be obtained.

According to one embodiment, the sequencing libraries are generated by using adapters and specific sequence motifs ("barcode") for library labeling and differentiation of the origin of the target sequence (i.e. the individual plant). Adapters comprising a barcode sequence may also be called "barcoded" or "index" adapters".

In some embodiments, the barcode sequence has a length of 4 or more nucleotides, in particular of between 4 and 8 nucleotides. In further embodiments, the barcode sequence is located between the target-specific hybridization sequence and the adapter sequence.

Typically, the size of the amplicons for sequencing ranges preferably from 100 bp to greater than 1000 bp depending on the length of the region one is amplifying and the DNA polymerase used. In some embodiments, the amplicon has a length/size between 100 and 1000 bp, in particular between 200 and 800 bp, in particular between 200 and 600 bp, in particular between 200 and 400 bp.

### Amplicon pooling

As mentioned above, the individual amplification products were pooled to prepare an amplicon library. Pooling the amplification products to create a library pool, multiple amplicon pools (amplicon library) may be combined in equimolar amounts to produce a library of amplicon pools which is used to construct a library for use in paired-end sequencing. For example, equimolar amounts from four 96-well amplicon pools targeting the same region of the target sequence may be combined to produce a 384-well amplicon pool to one region of the target sequence. Alternatively, a single 384-well plate is used to produce the 384-well amplicon pool. Equimolar amounts of a number of these 384-well amplicon pools targeting different regions of the target sequence or different target sequences may then be combined to produce a library pool. In one embodiment, five 384- well amplicon pools are combined to produce the library pool. The number of 384 well plates depends on the population size but can range from 1 to 15 384 well amplicon pools to produce a library pool.

### Sequencing

In a next step, the amplification products are sequenced to examine the target sequences of interests. As discussed above, sequencing is performed on a next generation sequencing platform. All NGS platforms share a common technological feature, namely the massively parallel sequencing e.g. of clonally amplified or single DNA molecules that are spatially separated in a flow cell or by generation of an oil-water emulsion. Massively parallel sequencing in particular refers to performing at least thousands (e.g. at least 50 000), at least 500 000 or at least 1 000 000 sequencing reactions in parallel per run. As described in the background, NGS allows thousands to billions of sequencing reactions to be performed simultaneously. In NGS, sequencing is performed by repeated cycles of polymerase-mediated nucleotide extensions or, in one common format, by iterative cycles of oligonucleotide ligation. After obtaining the target enriched sequencing library using the method according to the present invention, clonal separation of single molecules and subsequent amplification is performed by in vitro template preparation reactions like emulsion PCR (pyrosequencing from Roche 454, semiconductor sequencing from Ion Torrent, SOLiD sequencing by ligation from Life Technologies, sequencing by synthesis from Intelligent Biosystems), bridge amplification on the flow cell (e.g. Solexa/lllumina), isothermal amplification by Wildfire technology (Life Technologies) or rolonies/nanoballs generated by rolling circle amplification (Complete Genomics, Intelligent Biosystems, Polonator). Sequencing technologies like Heliscope (Helicos), SMRT technology (Pacific Biosciences) or nanopore sequencing (Oxford Nanopore) allow direct sequencing of single molecules without prior clonal amplification. Suitable NGS methods and platforms that can be used were also described in the background of the present invention and it is referred to the respective disclosure. The sequencing can be performed on any of the respective platforms using the target enriched sequencing library obtained according to the teachings of the present disclosure.

The obtained sequence information after sequencing may be aligned to provide the sequence of the target region. Here, methods known in the prior art can be used. Suitable methods are e.g. reviewed in Metzker, 2010.

The definitions and methods provided define the present invention and guide those of ordinary skill in the art in the practice of the present invention. Unless otherwise noted, terms are to be understood according to conventional usage by those of ordinary skill in the relevant art. Definitions of common terms in molecular biology may also be found in Alberts et al., Molecular Biology of The Cell, 5th Edition, Garland Science Publishing, Inc.: New York, 2007; Rieger et al., Glossary of Genetics: Classical and Molecular, 5th edition, Springer- Verlag: New York, 1991; King et al, A Dictionary of Genetics, 6th ed, Oxford University Press: New York, 2002; and Lewin, Genes IX, Oxford University Press: New York, 2007. The nomenclature for DNA bases as set forth at 37 CFR $ 1.822 is used.

### Identification

In another step, the sequenced target-sequence(s) may be compared with a known sequence (reference sequence), wherein the target-sequence can be allocated to the individual plant by the barcode sequence.

### Bioinformatic analysis

Processing and analysis of the NGS data has to be carried out by a Reference-Guided Genome Alignment Software suitable to handle NGS data rather than Sanger sequencing data. Typically the NGS sequencing data is trimmed and filtered of false reads and sequencing errors directly on the sequencing server and the optimized sequencing data is transferred into a file format which can be further analyzed with a customary, commercially available alignment software as e.g. Lasergene Genomic Suite (DNAStar, Madison, Wisconsin, USA). The optimized sequencing data has to be aligned to reference sequences for the different polymorphisms of the genomic regions of interest. In general the software for further analysis must be capable to handle multiple reference sequences in parallel and data from multiple distinct sequencing samples of similar or different genomic regions. Therefore multiple sequence alignments have to be carried out where multiple sequences can be aligned to a reference and to each other. Similar genomic regions from different samples have to be discriminated by barcode identification. Polymorphisms as SNPs or Indels have to be detected in the sequencing reads and visualized in an appropriate way. A statistical analysis of their distribution is necessary for a reliable detection of their zygosity in the respective sample.

### Plant Breeding

Plants that are screened and selected with a method of the present disclosure can be part of or generated from a breeding program. The choice of breeding method depends on the mode of plant reproduction, the heritability of the trait(s) being improved, and the type of cultivar used commercially (e.g., F1 hybrid cultivar, pure-line cultivar, etc.). A cultivar is a race or variety of a plant species that has been created or selected intentionally and maintained through cultivation.

Selected, non-limiting approaches for breeding the plants of the present invention are set forth below. A breeding program can be enhanced using marker assisted selection (MAS) on the progeny of any cross. It is understood that nucleic acid markers of the present invention can be used in a MAS (breeding) program. It is further understood that any commercial and non-commercial cultivars can be utilized in a breeding program. Factors such as, for example, emergence vigor, vegetative vigor, stress tolerance, disease resistance, branching, flowering, seed set, seed size, seed density, standability, and threshability etc. will generally dictate the choice.

For highly heritable traits, a choice of superior individual plants evaluated at a single location will be effective, whereas for traits with low heritability, selection should be based on mean values obtained from replicated evaluations of families of related plants. Popular selection methods commonly include pedigree selection, modified pedigree selection, mass selection, and recurrent selection. In a preferred aspect, a backcross or recurrent breeding program is undertaken. The complexity of inheritance influences choice of the breeding method.

Backcross breeding can be used to transfer one or a few favorable genes for a highly heritable trait into a desirable cultivar. This approach has been used extensively for breeding disease-resistant cultivars. Various recurrent selection techniques are used to improve quantitatively inherited traits controlled by numerous genes.

Breeding lines can be tested and compared to appropriate standards in environments representative of the commercial target area(s) for two or more generations.

The best lines are candidates for new commercial cultivars; those still deficient in traits may be used as parents to produce new populations for further selection.

For example, the methods of the present invention allow one skilled in the art to make plant breeding decisions regarding transgene modulating loci comprising: a) Selection among new breeding populations to determine which populations have the highest frequency of favorable haplotypes or genetic marker alleles, wherein haplotypes and marker alleles are designated as favorable based on coincidence with previous QTL mapping; or b) Selection of progeny containing the favorable haplotypes or genetic marker alleles in breeding populations prior to, or in substitution for, QTL mapping within that population, wherein selection could be done at any stage of breeding and could also be used to drive multiple generations of recurrent selection; or c) Prediction of progeny performance for specific breeding crosses; or d) S Selection of lines for germplasm improvement activities based on said favorable haplotypes or genetic marker alleles (as disclosed in PCT Patent Application Publication No. WO 2008/021413), including line development, hybrid development, selection among transgenic events based on the breeding value of the haplotype that the transgene is in linkage with (as disclosed in US Patent Application Serial No. 11/441,91), making breeding crosses, testing and advancing a plant through self-fertilization, purification of lines or sublines, using plant or parts thereof for transformation, using plants or parts thereof for candidates for expression constructs, and using plant or parts thereof for mutagenesis.

In addition, when the methods of the present invention are used for gene identification along with the use of integrated physical and genetic maps and various nucleic acid sequencing approaches, one skilled in the art can practice the combined methods to select for specific genes or gene alleles. For example, when haplotype windows are coincident with segments in which genes have been identified, one skilled in the art can extrapolate gene inferences to other germplasm having an identical genetic marker allele or alleles, or haplotype, in that haplotype window. This a priori information provides the basis to select for favorable genes or gene alleles on the basis of haplotype(s) or marker allele(s) identification within a given population.

The following examples are given to further illustrate the present invention without being deemed limitative thereof.

### Methods and Examples

In the following examples, materials and methods of the present disclosure are provided. It should be understood that these examples are for illustrative purpose only and are not to be construed as limiting this disclosure in any manner. All publications, patents, and patent applications cited herein are hereby incorporated by reference in their entirety for all purposes.

### 1. Detection of polymorphisms relevant for barley breeding

Using a multiplex PCR-based approach (Bentley, Higuchi, Hoglund, Goodridge, Sayer, Trachtenberg and Erlich, 2009, Fritsch, Fischer, Wambach, Dudek, Schillberg and Schröper, 2015) amplicons are generated representing naturally-occurring polymorphisms in two barley genes, namely the flowering time habit locus VrnH1 (Fu, Szucs, Yan, Helguera, Skinner, von Zitzewitz, Hayes and Dubcovsky, 2005, Szucs, Skinner, Karsai, Cuesta-Marcos, Haggard, Corey, Chen and Hayes, 2007, von Zitzewitz, Szucs, Dubcovsky, Yan, Francia, Pecchioni, Casas, Chen, Hayes and Skinner, 2005) and the grain protein content locus HvNAM1 (Cai, Yu, Chen, Huang, Jiang, Zhang and Jin, 2013, Uauy, Distelfeld, Fahima, Blechl and Dubcovsky, 2006) (Figure 1).

Each locus represents a relevant trait for breeding programs, VrnH1 in terms of adaptation to environmental conditions and HvNAM1 in terms of grain quality. The coverage of more than 1000 reads per PCR product ensured that the sequencing data were statistically valid and reduced the impact of sequencing errors, especially in the hybrids where anticipated polymorphisms only represented half of the reads. The assay was validated using two different genes containing different kinds of polymorphisms, i.e. Indels and single nucleotide polymorphisms (SNPs). As a result, the genotyping method according to the present disclosure is easy to implement, requires no PCR optimization steps and is suitable for the high-throughput analysis of many different samples and/or polymorphisms simultaneously.

### 1.1 Materials and methods

### a) Barley seeds, plant cultivation and interbreeding

Barley (Hordeum vulgare) seeds of the winter barley line Strider and the spring barley line Morex were used to prepare genomic templates representing the flowering time locus VrnH1. The Strider VrnH1 locus contains additional sequences that are not present in Morex, thus the locus is characterized by an Indel polymorphism (Figure 1 a).

Similarly, the Karl and Clipper lines were used to prepare genomic templates representing the grain protein content locus HvNAM1. Karl is a low grain protein content line with guanidine residues at three single nucleotide polymorphisms (SNPs) in this locus, whereas Clipper is a high grain protein content line with cytidine residues at the SNPs in the first two exons and an adenosine residue at the SNPs in exon three (Figure 1 b).

All seeds were provided by the National Small Grains Collection (Aberdeen, Idaho, USA) of the United States Department of Agriculture (USDA). To generate hybrids, the homozygous seeds were planted in Jiffy 7 pellets (Jiffy Products International BV, Moerdijk, Netherlands) and transferred to pots filled with Einheitserde classic substrate (Einheitserdewerke Werkverband e.V., Sinntal-Altengronau, Germany) after ~14 days. As soon as the awns started to grow out of the husks, the mother plant was emasculated by removing immature anthers from the flowers.

To interbreed the plants, ripe anthers from the paternal line were transferred to the emasculated flower and placed on the stigma of the maternal plant 2-3 days after the emasculation. The resulting hybrid seeds were harvested after ~3 months, when the seeds and plants were completely dry (Cornelia Marthe and Dr. Jochen Kumlehn, personal communication). To extract DNA, the homozygous and heterozygous seeds were planted in Jiffy-7 pellets and leaves from 7-14-day-old plants were processed with the Nucleospin Plant II kit (Machery-Nagel, Düren, Germany) according to the manufacturer's protocol. All DNA samples were dissolved in elution buffer (50 mM Tris-HCl, pH 8.5) to a final concentration of 150-250 ng/µl.

### b) Primers - general aspects

Primers were designed using CLC Main Workbench v6.9.1 (CLC Bio, Qiagen, Venlo, Netherlands) and synthesized by MWG-Biotech (Ebersberg, Germany). The primer sequences are listed in Table 2. The reference sequences for primer design were obtained from the NCBI nucleotide database (http://www.ncbi.nlm.nih.gov/nucleotide/). The barcodes and adapters associated with each primer are listed in Table 3 and 4.

**Table 2: PCR primers to generate NGS samples**

| **Oligo type** | **Target gene** | **Sequence (5'- 3')** | **Amplicon size (bp)** | **SEQ ID NO:** |
|---|---|---|---|---|
| FW primer | *VrnH1*_1 | ccagctgatgaaactccgaa | 234/244 | SEQ ID NO. 11 |
| RV primer | *VrnH1*_1_insertion | agcccatgtcagtttccagt | 234 | SEQ ID NO. 12 |
| RV primer | *VrnH1*_1_deletion | ggcgtatagtctcggagtga | 244 | SEQ ID NO. 13 |
| FW primer | *VrnH1*_2 | catttgcatctgccgatatg | 146/188 | SEQ ID NO. 14 |
| RV primer | *VrnH1*_2 | cttgaacggtatgagcgcta | 146/188 | SEQ ID NO. 15 |
| FW primer | *VrnH1*_3 | caagcaccacccaaccccaa | 251/268 | SEQ ID NO. 16 |
| RV primer | *VrnH1*_3 | tacagaaccgacgacccaag | 251/268 | SEQ ID NO. 17 |
| FW primer | *HvNAM1*_1 | ctcaagaagaaggccgccaa | 213 | SEQ ID NO. 18 |
| RV primer | *HvNAM1*_1 | tccacgcatccatccatcat | 213 | SEQ ID NO. 19 |
| FW primer | *HvNAM1*_2 | gtatgtgatcctgtcgtcgt | 236 | SEQ ID NO. 20 |
| RV primer | *HvNAM1*_2 | gagaaggtcggcgtcaagaa | 236 | SEQ ID NO. 21 |
| FW primer | *HvNAM1*_3 | taacaggagcagaaacgtcg | 214 | SEQ ID NO. 22 |
| RV primer | *HvNAM1*_3 | gtttccagcatcacgtccaa | 214 | SEQ ID NO. 23 |

| | | | | |
|---|---|---|---|---|
| bp: base pairs; FW: forward; RV: reverse | | | | |

**Table 3: Barcodes used to differentiate between the NGS reads**

| **Sequence (5' - 3')** | **Number** | **Target plant line** |
|---|---|---|
| ctcc | A01 | Karl |
| gcgt | A02 | Clipper |
| tgca | A03 | Karl x Clipper |
| acta | A04 | Morex |
| caga | A05 | Strider |
| aact | A06 | Morex x Strider |

**Table 4: Adapters**

| **Adapter type** | **Added to** | **Sequence (5'- 3')** | **SEQ ID NO:** |
|---|---|---|---|
| P1 | FW primer | cctctctatgggcagtcggtgat | SEQ ID NO. 24 |
| A | RV primer | ccatctcatccctgcgtgtctccgactcag | SEQ ID NO. 25 |

| | | | |
|---|---|---|---|
| FW: forward; RV: reverse | | | |

### c) Multiplex PCR to generate NGS templates

NGS templates were prepared by PCR on a Veriti 96-well thermocycler (Life Technologies, Carlsbad, USA) using the Expand High Fidelity PCR System (Roche, Rotkreuz, Switzerland), Axygen eight-strip tubes (Thermo Fisher Scientific, Waltham, USA) and eight-lid flat-cap strips (Sarstedt, Nümbrecht, Germany). Each reaction comprised 3.5 U Taq/Tgo DNA polymerase enzyme mix, 500 µM dNTPs, 0.5 µM of each primer (Table 2) and 150-200 ng of template DNA, topped up to 50 µl with the buffer supplied in the kit. The template was denatured at 95°C for 2 min and then amplified (30 cycles at 95°C for 30 s, 55°C for 30 s and 72°C for 30 s) followed by a final elongation step for 4 min at 72°C and indefinite storage at 8°C. PCR products were sized and quantified by capillary electrophoresis using the Agilent DNA 1000 Kit on an Agilent 2100 Bioanalyzer according to the manufacturer's instructions (Life Technologies). The PCR products were pre-diluted to at least 100 pM according to the concentration determined by capillary electrophoresis. The PCR products were diluted according to the concentration of the least concentrated amplicon because it was a multiplex reaction.

### d) Next-generation sequencing

The pre-diluted PCR products were purified and diluted to the final concentration of 26 pM using the Ion Library Equalizer Kit (Life Technologies). Therefore, 2-µl aliquots from each multiplex PCR vessel were pooled and topped up to 50 µl with elution buffer (50 mM Tris-HCl, pH 8.5). The pool was processed with the Ion Library Equalizer Kit using 90 µl Ampure beads and otherwise following the manufacturer's protocol. The pooled and equalized PCR products were then sequenced on an Ion Torrent Sequencer (Life Technologies) using an Ion 316 chip (Life Technologies). The results were analyzed using Lasergene Genomic Suite software (DNA Star, Madison, USA). The barcodes shown in Table 3 were used to differentiate among the samples.

With the example mentioned above a NGS-based polymorphism detection procedure was established using two previously described quantitative trait loci (QTLs), namely the barley flowering time locus VrnH1 (Fu, Szucs, Yan, Helguera, Skinner, von Zitzewitz, Hayes and Dubcovsky, 2005, Szucs, Skinner, Karsai, Cuesta-Marcos, Haggard, Corey, Chen and Hayes, 2007, von Zitzewitz, Szucs, Dubcovsky, Yan, Francia, Pecchioni, Casas, Chen, Hayes and Skinner, 2005) and the grain protein content locus HvNAM1 (Cai, Yu, Chen, Huang, Jiang, Zhang and Jin, 2013, Uauy, Distelfeld, Fahima, Blechl and Dubcovsky, 2006) (Figure 1).

Primers were designed to define amplicons of 146-268 bp spanning or flanking the polymorphisms of interest (Figure 2).

The 5.2-kb Indel polymorphism at the VrnH1 locus (Figure 1a) was amplified by competitive PCR with two reverse primers, one specific for the insert and one specific for the downstream 3' sequence flanking the insert (Figure 2 a).

Amplification of the entire insert with the forward and reverse flanking primers was prevented by limiting the PCR elongation time to 30 s, which is not enough to generate a full-size product of >5.4 kb because the polymerization rate of a standard PCR is approximately 1500 bp/min (Roche, 2011). The 42-bp and 17-bp Indels at the VrnH1 locus were amplified using primers flanking the Indels (Figure 2 b). The same strategy was used for the three SNPs at the HvNAM1 locus. Primers with barcodes (Table 3) and adapters (Table 4) were used to reduce the number of sample preparation steps by generating short PCR products, covering the Indels and SNPs, linked to two adapters and one sample-specific barcode that can be sequenced directly, without prior library preparation.

The preparation of a library involves fragmentation of the target DNA followed by end-repair, adapter ligation, purification and size selection (Figure 3) (Life Technologies, 2015, Thermo Fisher Scientific, 2014) and in our experience is time consuming and expensive, especially when processing a large number of samples.

Furthermore three PCRs were carried out to amplify the three polymorphisms at each locus (Figure 1) simultaneously in a multiplex reaction, rather than individually in three singleplex reactions, to limit the number of pipetting steps and therefore reduce consumables expenditure. Capillary electrophoresis was carried out using an Agilent 2100 Bioanalyzer to confirm the success of the reactions and to quantify the products. This is particularly important because the PCR products need to be diluted to exactly 26 pM for a successful sequencing (Life Technologies, 2013). Capillary electrophoresis showed that the PCRs were successful and bands with the anticipated sizes were observed. The samples were pre-diluted to 100 pM according to the capillary electrophoresis results. To reduce the number and cost of pipetting steps even further, 2 µl of each pre-diluted sample was pooled and processed collectively. The sample-pool was diluted to the required concentration, and thereby also purified, using a magnetic bead-based procedure with the Ion Library Equalizer Kit, therefore requiring on a single reaction tube and set of reagents per sample.

The reads generated by NGS were aligned to the VrnH1 and HvNAM1 reference sequences, allowing the genotypes to be clearly distinguished (Table 5a and 5b). For the VrnH1 gene, the three anticipated insertions were detected in all reads representing the Strider line, revealing the winter growth genotype of this QTL. The anticipated deletions were detected in all reads representing the Morex line, confirming the spring growth genotype of the QTL in this line. Insertions at these three locations correspond to winter barley alleles whereas deletions correspond to spring barley alleles (Fu, Szucs, Yan, Helguera, Skinner, von Zitzewitz, Hayes and Dubcovsky, 2005, Szucs, Skinner, Karsai, Cuesta-Marcos, Haggard, Corey, Chen and Hayes, 2007, von Zitzewitz, Szucs, Dubcovsky, Yan, Francia, Pecchioni, Casas, Chen, Hayes and Skinner, 2005). In the Morex x Strider hybrid, the insertions and deletions were distributed in approximately equal shares among the reads (Table 5a and 5b) and the loci could therefore be identified as heterozygous. The genotype of the 5.2-kb Indel could be detected by counting the reads aligned either to the 5' part of the insertion or to the downstream flanking sequence of the anticipated insertion (Figure 4) because competitive PCR was carried out with three primers (Figure 2 a).

The Strider line exclusively generated reads matching the 5' sequence of the insertion, whereas the Morex line exclusively generated reads matching the downstream flanking sequence. These results show that the Strider line contains the 5.2-kb insertion that is not present in the Morex line. The Morex x Strider hybrid generated reads aligning to both reference sequences, confirming that both Indel alleles are present. The 42-bp and 17-bp Indels were detected as gaps in the sequence. The sequencing results for the 17-bp Indel are shown as an example in Table 1 (Figure 5).

The three SNPs of interest in the HvNAM1 gene (Figure 1 b) were also detected by sequencing. The Clipper line contains guanidine residues at nucleotide positions 234 in the first exon and 544 in the second exon, whereas cytidine residues occupy both positions in the Karl line. In the third exon, the Clipper line contains a guanidine residue at nucleotide position 1433 whereas the Karl line contains an adenosine residue at this site. The Clipper alleles correspond to a high grain protein content, whereas the Karl alleles correspond to a low grain protein content phenotype (Cai, Yu, Chen, Huang, Jiang, Zhang and Jin, 2013, Uauy, Distelfeld, Fahima, Blechl and Dubcovsky, 2006). The Karl x Clipper hybrid showed a near equal distribution of the two alternative nucleotides in each position (Table 5a and 5b) confirming the heterozygosity of the hybrid at this locus. The sequencing results for the SNP at nucleotide position 234 are shown as an example in Table 1. An overview of the sequencing results and read numbers can be found in the Tables 5a and 5b.

The different numbers of reads aligned to the reference sequences of each locus (Figure 4, Table 5a and 5b in Figure 5 and 6) may reflect the uneven amplification efficiency of the multiplex PCR, which can be caused by differences in primer binding efficiency, the favored amplification of a specific target or the formation of primer dimers that inhibit amplification (Le, Hidalgo Ashrafi and Paul, 2009). Furthermore, read errors/low-quality reads are excluded from the final dataset. This often occurs when reads are automatically trimmed or filtered out, e.g. when they are polyclonal or produce an off-scale signal on the Ion Torrent server (Life Technologies, 2014). However, there was no need to normalize or equalize the PCRs in our method because a few reads are theoretically sufficient to confirm the presence of a given allele by mapping to a unique reference sequence. In heterozygous samples, those reads should be distributed in a near equal manner. Although specific limits have not been proposed, higher read numbers are known to reduce the error frequency significantly (Sims, Sudbery, Ilott, Heger and Ponting, 2014) and we therefore propose that a coverage of at least 30 reads per PCR product is desirable.

### References

The contents of all cited references, including literature references, issued patents, and published patent applications, as cited throughout this application are hereby expressly incorporated by reference.
Baldi, L., D. Hacker, et al. (2012). Large-Scale Transfection of Mammalian Cells. Protein Expression in Mammalian Cells. J. L. Hartley, Humana Press. 801: 13-26.
Chmiel, H. (2006). Bioprozesstechnik, ELSEVIER, Spektrum akademischer Verlag.
Derouazi, M., P. Girard, et al. (2004). "Serum-free large-scale transient transfection of CHO cells." Biotechnol Bioeng 87(4): 537-545.
Douglas, K. L. (2008). "Toward development of artificial viruses for gene therapy: a comparative evaluation of viral and non-viral transfection." Biotechnol Prog 24(4): 871-883.
Geisse, S., M. Jordan, et al. (2005). "Large-scale transient expression of therapeutic proteins in mammalian cells." Methods Mol Biol 308: 87-98.
Gursinsky, T., B. Schulz, et al. (2009). "Replication of Tomato bushy stunt virus RNA in a plant in vitro system." Virology 390(2): 250-260.
Hacker, D. L., E. Derow, et al. (2005). "The CELO adenovirus Gam1 protein enhances transient and stable recombinant protein expression in Chinese hamster ovary cells." J Biotechnol 117(1): 21-29.
Jordan, M. and F. Wurm (2004). "Transfection of adherent and suspended cells by calcium phosphate." Methods 33(2): 136-143.
Komoda, K., S. Naito, et al. (2004). "Replication of plant RNA virus genomes in a cell-free extract of evacuolated plant protoplasts." Proc Natl Acad Sci U S A 101(7): 1863-1867.
Pham, P., A. Kamen, et al. (2006). "Large-Scale transfection of mammalian cells for the fast production of recombinant protein." Mol Biotechnol 34(2): 225-237.
Sonobe, S. (1996). "Studies on the plant cytoskeleton using miniprotoplasts of tobacco BY-2 cells." J. Plant Res. 109(4): 437-448.

## Claims

1. A method for selecting a plant from a plant population by genotyping, the method comprises
a) isolating genomic DNA of individual plants or individual plant seeds separately, to provide separate DNA samples;
b) amplifying a desired target sequence of said DNA samples with target-specific primers, wherein each target-specific primer comprises a target-specific hybridization sequence and an adapter sequence, wherein at least one of the primers contains a barcode sequence, whereby the resulting amplification products (amplicons) comprise the target sequence, the adapter sequences and the barcode sequence;
c) pooling the amplicons of step (b) to prepare an amplicon library;
d) sequencing said amplified target sequence by using a next generation sequencing (NGS) technique;
e) comparing the target-sequence with a known sequence of said target-sequence, wherein the target-sequence can be allocated to an individual plant by the barcode sequence.

2. The method according to claim 1, wherein several different target sequences are amplified simultaneously.

3. The method according to one or more of claims 1 to 2, wherein the method comprises
a) isolating genomic DNA of individual plants or individual plant seeds separately, to provide separate DNA samples;
b) amplifying in parallel a plurality of different desired target sequences of said DNA samples with target-specific primers either separately or in a multiplex PCR reaction, wherein each target-specific primer comprises a target-specific hybridization sequence and an adapter sequence, wherein at least one of the primers contains a barcode sequence, whereby the resulting amplification products (amplicons) comprise the target sequence, the adapter sequences and the barcode sequence;
c) pooling the amplicons of step (b) to prepare an amplicon library;
d) sequencing said amplified target sequences by using a next generation sequencing (NGS) technique;
e) comparing the target-sequences with known sequences of said target-sequences, wherein the target-sequences can be allocated to an individual plant by the barcode sequences.

4. The method according to one or more of claims 1 to 3, wherein said target sequence is selected from the group consisting of a polynucleotide, a nucleic acid pattern and a genomic region, preferably the target sequence is a Quantitative Trait Loci (QTL).

5. The method according to one or more of claims 1 to 4, wherein a plurality of individual plants are genotyped in parallel, and wherein the genomic DNA of a plurality of individual plants are isolated and amplified without pooling the genomic DNA before amplifying.

6. The method according to one or more of claims 1 to 5, wherein the target sequence is a chromosomal segment that comprises a portion of a natural and/or artificial genetic rearrangement, wherein the genetic rearrangement is preferably an inversion, insertion, deletion, or translocation

7. The method according to one or more of claims 1 to 6, wherein the target sequence comprises a variation like a Single Nucleotide Polymorphism (SNP).

8. The method according to one or more of claims 1 to 7, wherein the target sequence comprises a mutation, or a target portion and/or a flanking portion

9. The method according to claim 8, wherein the target-specific hybridization sequence in the primer is complementary to a flanking portion.

10. The method according to claim 8, wherein the target-specific hybridization sequence is complementary to parts of the target portion.

11. The method according to one or more of claims 1 to 10, wherein the barcode sequence has a length of 4 or more nucleotides, in particular of between 4 and 8 nucleotides.

12. The method according to one or more of claims 1 to 11, wherein the barcode sequence is located between the target-specific hybridization sequence and the adapter sequence.

13. The method according to one or more of claims 1 to 12, wherein the amplicon has a length between 100 and 1000 bp, in particular between 200 and 800 bp, in particular between 200 and 600 bp, in particular between 200 and 400 bp.

14. The method according to one or more of claims 1 to 13, wherein the next generation sequencing method applied is selected from the group of sequencing by synthesis, pyrosequencing, ion semiconductor technology like Ion Torrent PGM, and single molecule real-time (SMRT™) sequencing.

15. The method according to one or more of claims 1 to 14, wherein the method is used for screening a plant and/or a plant population for multiple characteristics
